# EUROPÄISCHE PATENTSCHRIFT

(11) **EP 2 520 966 B1**
(45) Veröffentlichungstag und Bekanntmachung des Hinweises auf die Patenterteilung: **20.12.2017**
(21) Anmeldenummer: 12002769.3
(22) Anmeldetag: 20.04.2012
(51) Int. Cl.: A61B 1/00, G02B 23/24

(54) **Trockenmittelanordnung für optische Instrumente**
Desiccant assembly for optical instruments
Agencement de déshydratation pour instruments optiques

(30) Priorität: 02.05.2011 DE 102011100232
(43) Veröffentlichungstag der Anmeldung: 07.11.2012
(73) Patentinhaber: Karl Storz GmbH & Co. KG, 78532 Tuttlingen (DE)
(72) Erfinder: Bürk, Andre, 78054 Villingen-Schwenningen (DE); Egle, Michael, 78583 Böttingen (DE)
(74) Vertreter: Hofmeister, Frank

(56) Entgegenhaltungen:
- DE-A1- 2 128 428
- DE-A1- 19 507 205
- GB-A- 1 502 445
- US-A- 6 077 220
- US-A1- 2010 174 144

## Beschreibung

Die Erfindung betrifft eine Trockenmittelanordnung für optische Instrumente, insbesondere endoskopische Instrumente, mit einer im Instrumentengehäuse angeordneten hygroskopischen Substanz, wobei die hygroskopische Substanz als einzelne Formkörper ausgebildet, in einem auswechselbar in das Instrumentengehäuse einsetzbaren Aufnahmekörper angeordnet ist.

Optische Instrumente und insbesondere endoskopische Instrumente für medizinische und nichtmedizinische Zwecke sind prinzipiell fluiddichte Systeme. Durch eine Reihe von Gründen besteht aber die Möglichkeit, dass Feuchtigkeit in das Gehäuse eindringen kann, was zu einer die Sicht verschlechternden Trübung der optischen Systeme führen kann. Probleme können beispielsweise bereits bei der Fertigung der optischen Instrumente in normaler Atmosphäre entstehen, wenn sich die Restfeuchte der Atmosphärenluft im Gehäuseinneren niederschlägt. Weiterhin kann Feuchtigkeit durch geringfügige Undichtigkeiten an Verbindungsstellen eindringen, an denen das Instrument zu Wartungs-, Reparatur- oder Montagezwecken auseinander nehmbar ist. Eine weitere starke Belastung insbesondere medizinischer optischer Instrumente stellt beispielsweise das Reinigen mittels Autoklavieren dar, bei dem das Instrument unter wechselndem Druck Heißdampf bei etwa 140°C ausgesetzt wird. Diese Temperaturbelastung kann zu feinen Undichtigkeiten führen, durch die wiederum Feuchtigkeit in das Gehäuse eindringen kann.

Zur Vermeidung derartiger Beschlagprobleme durch sich auf dem optischen System niederschlagende Feuchtigkeit ist es bei optischen Instrumenten bekannt im Gehäuse eine hygroskopische Substanz anzuordnen, die die im Gehäuseinnenraum anfallende Feuchtigkeit bindet, bevor diese sich auf dem mindestens einen optischen System niederschlägt.

So ist es beispielsweise bekannt, die hygroskopische Substanz in loser Form im Gehäuse anzuordnen. Dies hat jedoch den Nachteil, dass beim Bewegen des Instruments Geräusche auftreten und zudem durch die Bewegung Abrieb der hygroskopischen Substanz erzeugt wird, der sich als Staub auf die optischen Systeme legen kann.

Aus der EP 0 916 106 B1 ist es weiterhin bekannt, die hygroskopische Substanz in der Form von vorgefertigten Formkörpern, wie beispielsweise Kugeln oder Stäbchen, in einer Ausnehmung in der Okularmuschel anzuordnen und mittels eines Fixierelements ortsfest in dieser Ausnehmung zu platzieren.

Diese bekannte Trockenmittelanordnung hat sich in der Praxis zwar durchaus bewährt, jedoch erlaubt die Anordnung der hygroskopischen Substanz in einer Gehäuseausnehmung nur den Einsatz einer geringen Trockenmittelmenge und Bedarf darüber hinaus einer speziellen Ausgestaltung der Okularmuschel.

Eine gattungsgemäße Trockenmittelanordnung ist beispielsweise aus der DE 103 44 109 A1 bekannt. Bei dieser bekannten Anordnung kann die hygroskopische Substanz beispielsweise in Kugelform ausgebildet sein, wobei diese kugeligen Formkörper zur Lagefixierung in einem Käfig angeordnet sind.

Davon ausgehend liegt der Erfindung die **Aufgabe** zugrunde, eine Trockenmittelanordnung zu schaffen, die bei einfacher vielseitiger Verwendung die Aufnahme einer großen Trockenmittelmenge ermöglicht.

Die **Lösung** dieser Aufgabenstellung ist erfindungsgemäß dadurch gekennzeichnet, dass der Aufnahmekörper als doppelwandige Hülse ausgebildet ist, wobei die beiden Wände einen Aufnahmeraum für die Formkörper der hygroskopischen Substanz bildend radial voneinander beabstandet so angeordnet sind, dass die Wände an einem axialen Ende der Hülse den Aufnahmeraum verschließend endseitig miteinander verbunden sind und am anderen axialen Ende der Hülse den Aufnahmeraum freigebend endseitig radial voneinander beabstandet sind.

Durch die erfindungsgemäße Anordnung der hygroskopischen Substanz in einem auswechselbar in das Instrumentengehäuse einsetzbaren Aufnahmekörper ist es möglich, die aus Aufnahmekörper und hygroskopischer Substanz bestehende Trockenmittelanordnung als eigenständige Baueinheit zu fertigen und zu bestücken, um diese fertige Baueinheit in einem einzigen Montageschritt im Instrumentengehäuse zu platzieren.

Durch die einseitig offene Ausgestaltung des Aufnahmeraums für die vorzugsweise als Kugeln oder Stäbchen ausgebildeten Formkörper der hygroskopischen Substanz wird bei einfacher und nur aus wenigen Bauteilen bestehender Bauweise eine Anordnung für das Trockenmittel geschaffen, die eine leichte Aufnahme eventuell in das Instrumentengehäuse eindringender Feuchtigkeit erlaubt.

Um die hygroskopische Wirkung der Trockenmittelanordnung zu vergrößern, wird mit der Erfindung vorgeschlagen, dass die Formkörper der hygroskopischen Substanz in axialer Richtung mehrlagig in dem Aufnahmekörper lagerbar sind, so dass bei einfacher Montage eine große Anzahl von Formkörper der hygroskopischen Substanz in dem Aufnahmekörper platzierbar sind.

Weiterhin wird mit der Erfindung vorgeschlagen, dass zur Aufnahme hygroskopischen Substanz im Aufnahmeraum radial umlaufend sich in axialer Richtung erstreckende separierte Aufnahmeschächte für die Formkörper der hygroskopischen Substanz ausgebildet sind, in denen die Formkörper der hygroskopischen Substanz radial voneinander beabstandet angeordnet sind. Diese Ausbildung der einzelnen Aufnahmeschächte im Aufnahmekörper ermöglicht einerseits ein einfaches und immer gleichmäßiges Bestücken des Aufnahmekörpers mit der hygroskopischen Substanz und verhindert andererseits, dass in radialer Richtung eine Relativbewegung zwischen den Formkörpern auftreten kann, die ihrerseits zu einem Staub bildenden Abrieb führen könnte.

Um die Formkörper der hygroskopischen Substanz ortsfest im Aufnahmekörper zu halten, wird erfindungsgemäß vorgeschlagen, dass die Formkörper der hygroskopischen Substanz über ein Fixierelement, vorzugsweise einen O-Ring, im Aufnahmekörper festlegbar sind.

Gemäß einer praktischen Ausführungsform der Erfindung wird vorgeschlagen, dass im Aufnahmekörper eine radial umlaufende Nut zur Aufnahme des Fixierelements ausgebildet ist, über die das Fixierelement einfach und schnell am Aufnahmekörper festlegbar ist.

Schließlich wird mit der Erfindung vorgeschlagen, dass die Innenseite der Hülse durch Farbgebung, Beschichtung und/oder Oberflächenstrukturierung so ausgebildet ist, dass das Auftreten von Streulicht möglichst gering gehalten werden kann. Zur Ausbildung einer Streulicht minimierenden Oberflächenstrukturierung sind auf der Innenseite der Hülse vorzugsweise Rillen mit Spitzen ausgebildet.

Weitere Merkmale und Vorteile der Erfindung ergeben sich anhand der zugehörigen Zeichnungen, in denen ein Ausführungsbeispiel einer erfindungsgemäßen Trockenmittelanordnung für optische Instrumente, nur beispielhaft dargestellt ist, ohne die Erfindung auf dieses Ausführungsbeispiel zu beschränken. In den Zeichnungen zeigt:
- Fig. 1: einen Längsschnitt durch die Okulareinheit eines optischen Instruments mit einer erfindungsgemäßen Trockenmittelanordnung;
- Fig. 2: eine Explosionszeichnung der Trockenmittelanordnung gemäß Fig. 1;
- Fig. 3: einen Längsschnitt durch die Trockenmittelanordnung gemäß Fig. 2 im montierten Zustand und
- Fig. 4: eine Draufsicht auf die Trockenmittelanordnung gemäß Fig. 3.

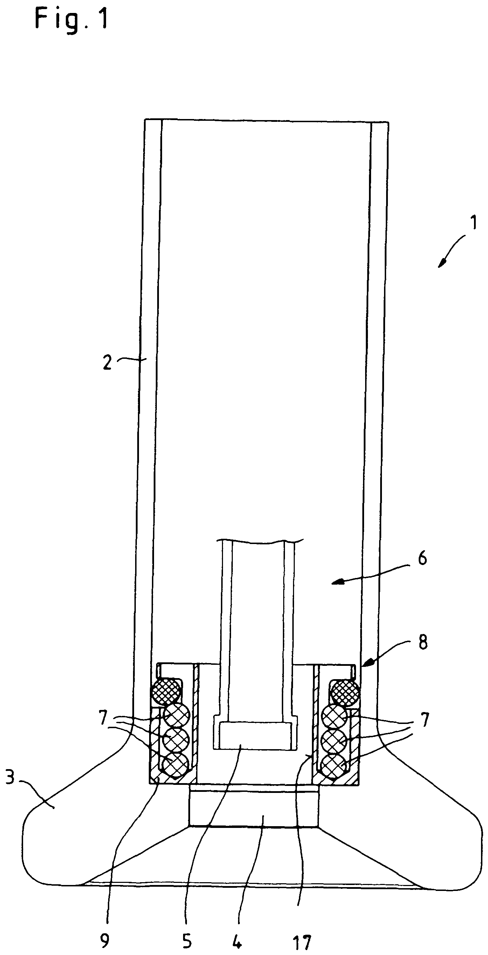

Die Abbildung Fig. 1 zeigt eine Okulareinheit 1 eines optischen Instruments, wie beispielsweise eines Endoskops für medizinische oder nichtmedizinische Zwecke.

Die dargestellte Okulareinheit 1 besteht im Wesentlichen aus einer Okularmuschelhülse 2 mit proximalseitig einstückig angeformter Okularmuschel 3 sowie einem Okulardeckglas 4, welches beispielsweise in die Okularmuschel 3 eingekittet, die Okulareinheit 1 proximalseitig fluiddicht verschließt. Im Inneren der Okulareinheit 1 ist ein mindestens eine Linse 5 aufweisendes optisches System 6 angeordnet. Distalseitig wird die einen Teil des Instrumentengehäuses 2 bildende Okularmuschelhülse 2 mit dem nicht dargestellten restlichen Instrumentengehäuse des Endoskops oder anderweitigen optischen Instruments fluiddicht verschraubt.

Alternativ zu der dargestellten einstückigen Ausbildung von Okularmuschel 3 und Okularmuschelhülse 2 ist es selbstverständlich auch möglich, die Okularmuschel 3 und die Okularmuschelhülse 2 als, beispielsweise durch Verschrauben, miteinander verbindbare separate Bauteile auszubilden.

Zum Entfernen der eventuell im Instrumentengehäuse 2 vorhandenen Restfeuchte sowie zur Aufnahme von über Undichtigkeiten in das Instrumentengehäuse 2 eingedrungener Feuchtigkeit ist im Inneren des Instrumentengehäuses 2 eine mit einer hygroskopischen Substanz 7 bestückte Trockenmittelanordnung 8 angeordnet. Als Materialien für die Herstellung der hygroskopischen Substanz 7 werden beispielsweise synthetische Zeolithe oder andere Tonminerale, oder aber auch SilikaGel verwendet.

Die in den Abbildungen Fig. 1 bis 4 dargestellte Trockenmittelanordnung 8 besteht aus einem in die Okularmuschelhülse 2 auswechselbar einsetzbaren Aufnahmekörper 9, in dem die als Formkörper ausgebildete hygroskopische Substanz 7 angeordnet ist. Bei der dargestellten Ausführungsform sind die Formkörper der hygroskopischen Substanz 7 als Kugeln ausgebildet. Ebenso können die Formkörper der hygroskopischen Substanz 7 stabförmig ausgebildet sein.

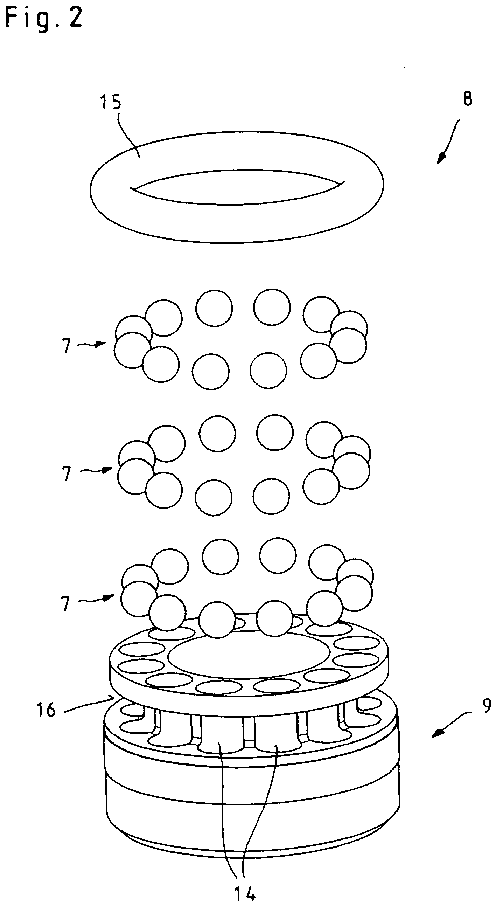

Der Aufbau der Trockenmittelanordnung 8 ist insbesondere den Abbildungen Fig. 2 bis 4 zu entnehmen.

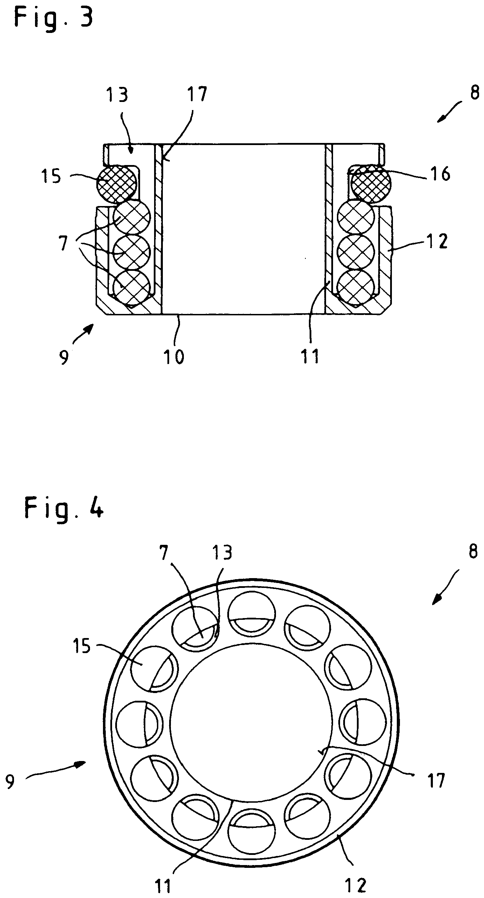

Wie insbesondere aus Fig. 3 ersichtlich, ist der Aufnahmekörper 9 als doppelwandige Hülse 10 ausgebildet, wobei die beiden Wände 11 und 12 der doppelwandigen Hülse 10 einen Aufnahmeraum 13 für die Formkörper der hygroskopischen Substanz 7 bildend radial voneinander beabstandet koaxial zueinander angeordnet sind. Die Hülse 10 ist so ausgebildet, dass die Wände 11 und 12 an einem axialen Ende der Hülse 10 den Aufnahmeraum 13 verschließend endseitig miteinander verbunden sind, während sie am anderen axialen Ende der Hülse 10 den Aufnahmeraum 13 freigebend endseitig radial voneinander beabstandet sind.

Im zur Aufnahme der hygroskopischen Substanz 7 dienenden Aufnahmeraum 13 sind radial umlaufend sich in axialer Richtung erstreckende separierte Aufnahmeschächte 14 für die Formkörper der hygroskopischen Substanz 7 ausgebildet, in denen die Formkörper der hygroskopischen Substanz 7 radial voneinander beabstandet angeordnet sind. Diese Ausbildung der einzelnen Aufnahmeschächte 14 im Aufnahmekörper 13 ermöglicht einerseits ein einfaches und immer gleichmäßiges Bestücken des Aufnahmekörpers 9 mit der hygroskopischen Substanz 7 und verhindert andererseits, dass in radialer Richtung eine Relativbewegung zwischen den Formkörpern auftreten kann, die ihrerseits zu einem Staub bildenden Abrieb führen könnte.

Bei der dargestellten Ausführungsform des Aufnahmekörpers 9 können drei Lagen zu jeweils zwölf kugelförmigen Formkörpern der hygroskopischen Substanz 7 in den Aufnahmeschächten 14 des Aufnahmekörpers 9 angeordnet werden.

Um die Formkörper der hygroskopischen Substanz 7 ortsfest im Aufnahmekörper 9 zu halten, ist vorzugsweise als O-Ring ausgebildetes Fixierelement 15 vorgesehen, das in eine im Aufnahmekörper 9 ausgebildete radial umlaufende Nut 16 einsetzbar ist. Bei der dargestellten Ausführungsform ist die radial umlaufende Nut 16 in der radial äußeren Wand 12 der Hülse 10 so ausgebildet und angeordnet, dass das in die Nut 16 eingesetzte, vorzugsweise als O-Ring ausgebildete Fixierelement 15 im montierten Zustand in radialer Richtung nach außen im Wesentlichen bündig mit der Außenseite der Wand 12 der Hülse 10 abschließt und in radialer Richtung nach innen in etwa nur die halbe radiale Tiefe der Aufnahmeschächte 14 abdeckt, um bei sicherem Halt einen möglichst großen freien Zugang zur hygroskopischen Substanz zu gewährleisten.

In axialer Richtung ist die Nut 16 so in der Wand 12 der Hülse 10 ausgebildet, dass das in die Nut 16 eingesetzte Fixierelement 15 im montierten Zustand am obersten Formkörper der hygroskopischen Substanz 7 anliegt, um so die einzelnen in den Aufnahmeschächten 14 angeordneten Formkörper der hygroskopischen Substanz 7 in axialer Richtung gegeneinander zu fixieren.

Durch die Anordnung der hygroskopischen Substanz 7 in dem auswechselbar in das Instrumentengehäuse 2 einsetzbaren Aufnahmekörper 9 ist es möglich, die aus Aufnahmekörper 9 und hygroskopischer Substanz 7 bestehende Trockenmittelanordnung 8 als eigenständige Baueinheit zu fertigen und zu bestücken, um diese fertige Baueinheit in einem einzigen Montageschritt, beispielsweise durch Einpressen, im Instrumentengehäuse 2 zu platzieren.

Die Ausbildung des separaten Aufnahmekörpers 9 hat weiterhin den Vorteil, dass die Länge der Okularmuschelhülse 2 beliebig wählbar ist, da es keiner Anpassung der Okularmuschelhülse 2 an den Aufnahmekörper 2 bedarf. Der Aufbau des Aufnahmekörpers 9 stellt weiterhin sicher, dass die hygroskopische Substanz 7 durch kein anderes Medium benetzt werden kann und die offene Bauweise eine gute Aufnahme eventuell in die Okularmuschelhülse 2 eindringender Feuchtigkeit durch die im Aufnahmekörper 9 angeordnete hygroskopische Substanz gewährleistet.

Um das Auftreten von Streulicht im Inneren der Okularmuschelhülse 2 möglichst gering halten zu können, kann die Innenseite 17 der Hülse 10 durch Farbgebung, Beschichtung und/oder Oberflächenstrukturierung so ausgebildet werden, dass die Gefahr einer Beeinträchtigung des optischen Systems 6 minimiert wird. Zur Ausbildung einer Streulicht minimierenden Oberflächenstrukturierung können auf der Innenseite 17 der Hülse 10 vorzugsweise mit Spitzen versehene Rillen ausgebildet sein.

### Bezugszeichenliste

- 1: Okulareinheit
- 2: Okularmuschelhülse / Instrumentengehäuse
- 3: Okularmuschel
- 4: Okulardeckglas
- 5: Linse
- 6: optisches System
- 7: hygroskopische Substanz
- 8: Trockenmittelanordnung
- 9: Aufnahmekörper
- 10: Hülse
- 11: Wand
- 12: Wand
- 13: Aufnahmeraum
- 14: Aufnahmeschacht
- 15: Fixierelement
- 16: Nut
- 17: Innenseite

## Patentansprüche

1. Trockenmittelanordnung für optische Instrumente, insbesondere endoskopische Instrumente, mit einer im Inneren des Instrumentengehäuses (2) des optischen Instruments angeordneten hygroskopischen Substanz (7), wobei die hygroskopische Substanz (7) als einzelne Formkörper ausgebildet, in einem auswechselbar in das Instrumentengehäuse (2) einsetzbaren Aufnahmekörper (9) angeordnet ist,
**dadurch gekennzeichnet,**
**dass** der Aufnahmekörper (9) als doppelwandige Hülse (10) ausgebildet ist, wobei die beiden Wände (11 und 12) einen Aufnahmeraum (13) für die Formkörper der hygroskopischen Substanz (7) bildend radial voneinander beabstandet so angeordnet sind, dass die Wände (11 und 12) an einem axialen Ende der Hülse (10) den Aufnahmeraum (13) verschließend endseitig miteinander verbunden sind und am anderen axialen Ende der Hülse (10) den Aufnahmeraum (13) freigebend endseitig radial voneinander beabstandet sind.

2. Trockenmittelanordnung nach Anspruch 1, **dadurch gekennzeichnet, dass** die Formkörper der hygroskopischen Substanz (7) in axialer Richtung mehrlagig in dem Aufnahmekörper (9) lagerbar sind.

3. Trockenmittelanordnung nach Anspruch 1 oder 2, **dadurch gekennzeichnet, dass** im Aufnahmeraum (13) radial umlaufend sich in axialer Richtung erstreckende separierte Aufnahmeschächte (14) für die Formkörper der hygroskopischen Substanz (7) ausgebildet sind.

4. Trockenmittelanordnung nach Anspruch 3, **dadurch gekennzeichnet, dass** die Formkörper der hygroskopischen Substanz (7) radial voneinander beabstandet in den Aufnahmeschächten (14) des Aufnahmeraums (13) angeordnet sind.

5. Trockenmittelanordnung nach einem der Ansprüche 1 bis 4, **dadurch gekennzeichnet, dass** die Formkörper der hygroskopischen Substanz (7) über ein Fixierelement (15), vorzugsweise einen O-Ring, im Aufnahmekörper (9) festlegbar sind.

6. Trockenmittelanordnung nach Anspruch 5, **dadurch gekennzeichnet, dass** im Aufnahmekörper (9) eine radial umlaufende Nut (16) zur Aufnahme des Fixierelements (15) ausgebildet ist.

7. Trockenmittelanordnung nach einem der Ansprüche 1 bis 6, **dadurch gekennzeichnet, dass** die Formkörper der hygroskopischen Substanz (7) als Kugeln oder Stäbchen ausgebildet sind.

8. Trockenmittelanordnung nach einem der Ansprüche 1 bis 7, **dadurch gekennzeichnet, dass** die Innenseite (17) der Hülse (10) durch Farbgebung, Beschichtung und/oder Oberflächenstrukturierung Streulicht minimierend ausgestaltet ist.

9. Trockenmittelanordnung nach Anspruch 5, **dadurch gekennzeichnet, dass** auf der Innenseite (17) der Hülse (10) zur Ausbildung einer Streulicht minimierenden Oberflächenstruktur Rillen mit Spitzen ausgebildet sind.

## Claims

1. A desiccant assembly for optical instruments, in particular endoscopic instruments, with a hygroscopic substance (7) arranged in the interior of the instrument housing (2) of the optical instrument, wherein the hygroscopic substance (7) formed as individual moulded bodies is arranged in a receiving body (9) which can be inserted exchangeably into the instrument housing (2),
**characterised in that** the receiving body (9) is formed as a double-walled sleeve (10), wherein the two walls (11 and 12) forming a receiving space (13) for the moulded bodies of the hygroscopic substance (7) are arranged spaced apart from one another, so that the walls (11 and 12) are connected to one another at the end on one axial end of the sleeve (10), closing the receiving space (13), and are radially spaced apart from one another at the end on the other axial end of the sleeve (10), releasing the receiving space (13).

2. The desiccant assembly according to claim 1, **characterised in that** the moulded bodies of the hygroscopic substance (7) can be stored in axial direction in multiple layers in the receiving body (9).

3. The desiccant assembly according to claim 1 or 2, **characterised in that**, in receiving space (13), separated receiving shafts (14) extending radially peripherally in axial direction are formed for the moulded bodies of the hygroscopic substance (7).

4. The desiccant assembly according to claim 3, **characterised in that** the moulded bodies of the hygroscopic substance (7) are arranged radially spaced apart from one another in the receiving shafts (14) of the receiving space (13).

5. The desiccant assembly according to any one of claims 1 to 4, **characterised in that** the moulded bodies of the hygroscopic substance (7) can be immobilised via a fastening element (15), preferably an O-ring, in the receiving body (9).

6. The desiccant assembly according to claim 5, **characterised in that**, in the receiving body (9), a radially peripheral groove (16) for receiving the fastening element (15) is formed.

7. The desiccant assembly according to any one of claims 1 to 6, **characterised in that** the moulded bodies of the hygroscopic substance (7) are formed as beads or rods.

8. The desiccant assembly according to any one of claims 1 to 7, **characterised in that** the inner side (17) of the sleeve (10) is designed to minimize scattered light by colouring, coating and/or surface structuring.

9. The desiccant assembly according to claim 5, **characterised in that** grooves with points are formed on the inner side (17) of the sleeve (10) for the formation of a surface structure which minimizes scattered light.

## Revendications

1. Agencement d'agent dessiccant pour instruments optiques, notamment des instruments endoscopiques, comprenant une substance hygroscopique (7) agencée à l'intérieur du boitier d'instrument (2) de l'instrument optique, la substance hygroscopique (7) étant d'une configuration sous forme de corps de forme individuels et agencée dans un corps d'accueil (9) pouvant être inséré de manière interchangeable dans le boitier d'instrument (2),
**caractérisé**
**en ce que** le corps d'accueil (9) est réalisé sous la forme d'un fourreau à double paroi (10), les deux parois (11 et 12), en formant un compartiment d'accueil (13) pour les corps de forme de substance hygroscopique (7), étant agencées de manière à être espacées radialement l'une de l'autre de façon à ce que les parois (11 et 12) soient reliées l'une à l'autre à leur bout au niveau d'une extrémité axiale du fourreau (10), en fermant le compartiment d'accueil (13), et soient espacées radialement l'une de l'autre à leur bout au niveau de l'autre extrémité axiale du fourreau (10), en permettant l'accès libre au compartiment d'accueil (13).

2. Agencement d'agent dessiccant selon la revendication 1, **caractérisé en ce que** les corps de forme de la substance hygroscopique (7) peuvent être placés selon plusieurs couches, en direction axiale, dans le corps d'accueil (9).

3. Agencement d'agent dessiccant selon la revendication 1 ou la revendication 2, **caractérisé en ce que** dans le compartiment d'accueil (13) sont formés, de manière radialement périphérique, des puits d'accueil (14) séparés, qui s'étendent dans la direction axiale et sont destinés aux corps de forme de la substance hygroscopique (7).

4. Agencement d'agent dessiccant selon la revendication 3, **caractérisé en ce que** les corps de forme de la substance hygroscopique (7) sont agencés de manière radialement espacée les uns des autres dans les puits d'accueil (14) du compartiment d'accueil (13).

5. Agencement d'agent dessiccant selon l'une des revendications 1 à 4, **caractérisé en ce que** les corps de forme de la substance hygroscopique (7) peuvent être fixés dans le corps d'accueil (9) par l'intermédiaire d'un élément de fixation (15), de préférence un joint torique.

6. Agencement d'agent dessiccant selon la revendication 5, **caractérisé en ce que** dans le corps d'accueil (9) est réalisée une rainure (16) radialement périphérique et destinée à accueillir l'élément de fixation (15).

7. Agencement d'agent dessiccant selon l'une des revendications 1 à 6, **caractérisé en ce que** les corps de forme de la substance hygroscopique (7) sont réalisés sous forme de billes ou de petits barreaux.

8. Agencement d'agent dessiccant selon l'une des revendications 1 à 7, **caractérisé en ce que** le côté intérieur (17) du fourreau (10) est d'une configuration minimisant la lumière diffusée ou parasite, en étant coloré, en étant pourvu d'un revêtement et/ou en présentant une structuration de surface.

9. Agencement d'agent dessiccant selon la revendication 5, **caractérisé en ce que** sur le côté intérieur (17) du fourreau (10) sont réalisées des stries avec des pointes pour former une structure de surface minimisant la lumière diffusée ou parasite.
